# EUROPEAN PATENT APPLICATION

(11) **EP 4 557 613 A1**
(43) Date of publication of application: **21.05.2025**
(21) Application number: 23838413.5
(22) Date of filing: 22.02.2023
(51) Int. Cl.: H03K 3/01

(54) **PULSE GENERATION CIRCUIT, PULSE GENERATOR, AND MEDICAL DEVICE**

(30) Priority: 12.07.2022 CN 202210816930
(71) Applicant: Shenzhen Pulsecare Medical Technology Co., Ltd., Shenzhen, Guangdong 518000 (CN)
(72) Inventor: TAN, Jianwen, Shenzhen, Guangdong 518000 (CN); XU, Li, Shenzhen, Guangdong 518000 (CN)
(74) Representative: Zaboliene, Reda
(86) International application number: PCT/CN2023/077704
(87) International publication number: WO 2024/011911

(57) **Abstract**

The present application relates to a pulse generation circuit, a pulse generator, and a medical device. The pulse generation circuit (10) includes: a control circuit (100), a high-voltage power supply circuit (210), a working power supply circuit (220), a pulse switch circuit (500), a plurality of photoelectric isolation drive circuits (300) and a plurality of magnetic isolation power supply circuits (400). According to the present application, isolation between various power switches (510) can be realized by means of the above photoelectric isolation drive circuits and magnetic isolation power supply circuits; moreover, the photoelectric isolation drive circuits have higher response speeds, such that the synchronization rate of the respective power switches can be improved by controlling the power switches according to a switch control signal by means of the photoelectric isolation drive circuits.

## Description

The present application claims priority of the Chinese Patent Application No. 202210816930.9 entitled "Pulse Generation Circuit, Pulse Generator and Medical Device" and filed with China National Intellectual Property Administration on July 12, 2022, the entire contents of which are incorporated herein by reference.

### TECHNICAL FIELD

The present invention relates to the technical field of signal generators, and in particular relates to a pulse generation circuit, a pulse generator and a medical device.

### BACKGROUND

At present, high-voltage pulse is a form of high-voltage transient energy that is narrow in time domain and wide in frequency domain. Due to its characteristics of high voltage, high peak power, and rich frequency components, it has been widely used in fields such as industrial industry, medical industry, military industry, and testing.

Also due to the above characteristics, the generation, measurement, control, protection, and application of high-voltage pulses are difficult. With regard to the generation of high-voltage pulses, the core problem lies in the high-voltage pulse discharge switches. For high-repetition-frequency high-voltage pulses, the use of all-solid-state semiconductor switch devices is the mainstream choice under the current domestic and international technical level and development trend. The commonly used pulse forming circuit methods currently include three methods: multiple switch devices in series and parallel, voltage superposition, and a combination of different switch devices.

The solution of multiple switch devices in series and parallel has the characteristics of good versatility and high modularity, but it requires to solve the problem of synchronous control and circuit isolation for the connection of switch devices in series. Due to the great differences in the consistency of various switch devices and other components, it is difficult to ensure synchronization during high-speed switching.

### SUMMARY

### Technical problem

The objective of this application is to provide a pulse generation circuit, a pulse generator and a medical device, aiming to solve the problems of synchronous control and circuit isolation between multiple switch devices existing in traditional pulse generation circuits.

### Technical solutions

A first aspect of embodiments of the present application provides a pulse generation circuit, including: a control circuit, a high-voltage power supply circuit, a working power supply circuit, a pulse switch circuit, a plurality of photoelectric isolation drive circuits and a plurality of magnetic isolation power supply circuits; wherein the pulse switch circuit includes a plurality of power switches connected in series between the high-voltage power supply circuit and a ground terminal, and the pulse switch circuit is configured to generate and output a high-voltage pulse signal by turning on or off the plurality of the power switches based on a driving voltage provided by the high-voltage power supply circuit; wherein the photoelectric isolation drive circuits are equal in number to the power switches and correspond to the power switches in a one-to-one corresponding relationship, each of the photoelectric isolation drive circuits is individually connected between a corresponding power switch and the control circuit, and the photoelectric isolation drive circuit is configured to control the turning on or off of the corresponding power switch according to a received switch control signal output by the control circuit; and wherein the magnetic isolation power supply circuits are equal in number to the photoelectric isolation drive circuits and correspond to the photoelectric isolation drive circuits in a one-to-one corresponding relationship, each of the magnetic isolation power supply circuits is individually connected to a corresponding photoelectric isolation drive circuit, each of the magnetic isolation power supply circuits is connected in series between the working power supply circuit and the ground terminal, and the magnetic isolation power supply circuit is configured to supply power to the corresponding photoelectric isolation drive circuit based on a working voltage provided by the working power supply circuit.

In one embodiment, each of the photoelectric isolation drive circuits includes a photoelectric coupling unit, the photoelectric coupling units are equal in number to the power switches and corresponds to the power switches in a one-to-one corresponding relationship, an input end of each of the photoelectric coupling units is connected to the control circuit, an output end of each of the photoelectric coupling units is individually connected to the corresponding power switch, and the photoelectric coupling unit is configured to unidirectionally transmit the switch control signal to the corresponding power switch through photoelectric conversion.

In one embodiment, each of the photoelectric isolation drive circuits further includes a drive unit, the driving unit are equal in number to the photoelectric coupling units and corresponds to the photoelectric coupling units in a one-to-one corresponding relationship, each of the drive units is individually connected between the output end of a corresponding photoelectric coupling unit and the corresponding power switch, and each drive unit is configured to output a corresponding voltage level to the corresponding power switch according to the switch control signal output by the corresponding photoelectric coupling unit to control the turning on or off of the power switch.

In one embodiment, each of the photoelectric isolation drive circuits further includes a plurality of delay units, the delay units are equal in number to the photoelectric coupling units and correspond to the photoelectric coupling units in a one-to-one corresponding relationship, and each of the delay units is individually connected between the input end of a corresponding photoelectric coupling unit and the control circuit to adjust a time for the switch control signal to be transmitted to the photoelectric coupling unit.

In one embodiment, each of the magnetic isolation power supply circuits includes a transformer and a rectifier unit, the transformers are equal in number to the photoelectric coupling units and correspond to the photoelectric coupling units in a one-to-one corresponding relationship, the rectifier units are equal in number to the photoelectric coupling units and correspond to the photoelectric coupling units in a one-to-one corresponding relationship. A primary winding of each transformer is connected in series between the working power supply circuit and the ground terminal, and a secondary winding of each transformer is individually connected to a corresponding rectifier unit, and each rectifier unit is individually connected to the corresponding photoelectric isolation drive circuit to output a corresponding working voltage to the corresponding photoelectric isolation drive circuit.

In one embodiment, the pulse switch circuit further includes a plurality of voltage equalizing circuits, the voltage equalizing circuits are equal in number to the power switches and correspond to the power switches in a one-to-one corresponding relationship. Each voltage equalizing circuit is individually connected in parallel with a corresponding power switch to adjust a voltage across a corresponding power switch.

In one embodiment, the voltage equalizing circuit includes a static voltage equalizing resistor; a first end of the static voltage equalizing resistor is connected to a first conducting end of the corresponding power switch, and a second end of the static voltage equalizing resistor is connected to a second conducting end of the corresponding power switch.

In one embodiment, each voltage equalizing circuit includes a dynamic voltage equalizing resistor and a dynamic voltage equalizing capacitor; a first end of the dynamic voltage equalizing resistor is connected to the first conducting end of the corresponding power switch, a second end of the dynamic voltage equalizing resistor is connected to a first end of the dynamic voltage equalizing capacitor, and a second end of the dynamic voltage equalizing capacitor is connected to the second conducting end of the corresponding power switch.

In one embodiment, the pulse generation circuit further includes a current detection circuit, wherein the current detection circuit is connected between the high-voltage power supply circuit and the pulse switch circuit and connected to the control circuit. The current detection circuit is configured to detect a current output from the high-voltage power supply circuit to the pulse switch circuit, generate a current sampling signal and feed it back to the control circuit; and wherein the control circuit is connected to the high-voltage power supply circuit and is configured to control the high-voltage power supply circuit to be turned off when the current sampling signal exceeds a preset safety range.

A second aspect of embodiments of the present application provides a pulse generator, including two pulse generation circuits as described above, for outputting a bipolar high-voltage pulse signal through the two pulse generation circuits under the control of the control circuit.

A third aspect of embodiments of the present application provides a medical device, including an ablation electrode needle and a pulse generator as described above, wherein the ablation electrode needle is connected to the pulse generator to release the bipolar high-voltage pulse signal.

### Technical effects

Compared with the prior art, the embodiments of the present application have the following technical effects: the present application can achieve isolation among the power switch, the high-voltage power supply circuit, the working power supply circuit and the control circuit through the above-mentioned photoelectric isolation drive circuit and magnetic isolation power supply circuit, and can especially achieve isolation between the high-voltage power supply circuit and other circuits, so that each power switch can be independently controlled and not affected by the high-voltage power supply circuit. At the same time, the photoelectric isolation drive circuit has a faster response speed, and the power switch is controlled by the photoelectric isolation drive circuit according to the switch control signal, which can improve the synchronization rate of the respective power switches while achieving electrical isolation.

### BRIEF DESCRIPTION OF DRAWINGS

In order to illustrate more clearly the technical solutions in the embodiments of the present application, the drawings needed to be used in the embodiments or description of the prior art will be briefly introduced below. Apparently, the drawings in the following description are only some embodiments of the present application. For the skilled persons in the art, other drawings can be obtained based on these drawings without exerting creative efforts.
FIG. 1 is a schematic diagram of the principle of the pulse generation circuit provided in the first embodiment of the present application;
FIG. 2 is a schematic diagram of the principle of the photoelectric isolation drive circuit provided in the first embodiment of the present application;
FIG. 3 is a schematic diagram of the principle of the photoelectric isolation drive circuit provided in another embodiment of the present application;
FIG. 4 is a schematic diagram of the circuitry of the magnetic isolation power supply circuit provided in the first embodiment of the present application;
FIG. 5 is a schematic diagram of the connection relationship between the magnetic isolation power supply circuit and the photoelectric isolation drive circuit provided in the first embodiment of the present application;
FIG. 6 is a schematic diagram of the principle of the pulse switch circuit provided in the first embodiment of the present application;
FIG. 7 is a schematic diagram of the circuit of the voltage equalizing unit provided in the first embodiment of the present application;
FIG. 8 is a schematic diagram of the principle of the pulse generation circuit provided in another embodiment of the present application;
FIG. 9 is a schematic diagram of the principle of the current detection circuit provided in another embodiment of the present application;
FIG. 10 is a schematic diagram of the principle of the pulse generator provided in the second embodiment of the present application;
FIG. 11 is a schematic diagram of the principle of the medical device provided in the third embodiment of the present application.

Descriptions of the reference numerals in the above drawings:
10, pulse generation circuit; 20, pulse generator; 30, ablation electrode needle; 100, control circuit; 210, high-voltage power supply circuit; 220, working power supply circuit; 300, photoelectric isolation drive circuit; 310, photoelectric coupling unit; 320, drive unit; 330, delay unit; 400, magnetic isolation power supply circuit; 410, transformer; 420, rectifier unit; 430, voltage regulating unit; 500, pulse switch circuit; 510, power switch; 520, first switch branch; 530, second switch branch; 540, voltage equalizing unit; 600, current detection circuit; 610, comparison unit.

### DESCRIPTION OF EMBODIMENTS

In order to make the purpose, technical solutions and advantages of the present application clearer, the present application will be further described in detail below with reference to the accompanying drawings, namely embodiments. It should be understood that the specific embodiments described here are only used to explain the present application and are not used to limit the present application.

It should be noted that when an element is referred to as being "fixed to" or "disposed on" another element, it can be directly or indirectly provided on the other element. When an element is referred to as being "connected to" another element, it may be directly or indirectly connected to the other element. The orientation or positional relationship indicated by the terms "upper", "lower", "left", "right", etc. are based on the orientation or positional relationship shown in the drawings. They are only for convenience of describing the present application and do not indicate or imply the device or element to which they are referred must have a specific orientation, or be constructed and operated in a specific orientation, and therefore cannot be construed as a limitation on the patent. The terms "first" and "second" are only used for descriptive purposes and cannot be understood as indicating or implying relative importance or implicitly indicating the quantity of technical features. "A plurality of" means two or more, unless otherwise clearly and specifically defined.

It should also be noted that in the embodiments of the present application, the same reference numerals refer to the same elements or components. For the same elements in the embodiments of the present application, only one of the elements or components may be labeled in the figure as an example. It should be understood that the same reference numerals are applicable to other identical elements or components.

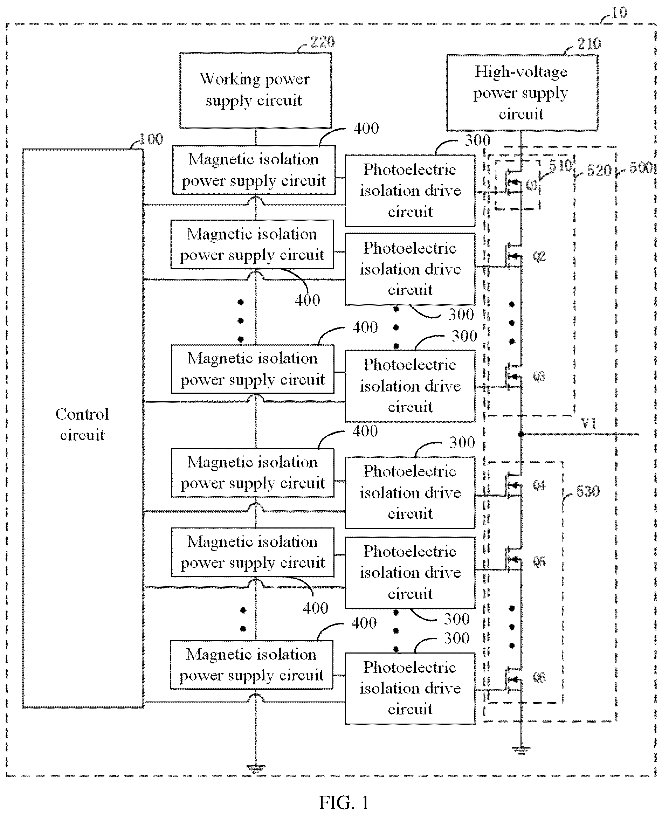

FIG. 1 shows a schematic diagram of the principle of a pulse generation circuit provided in the first embodiment of the present application. For convenience of explanation, only the parts related to the present embodiment are shown. The details are described as follows.

A pulse generation circuit 10 includes: a control circuit 100, a high-voltage power supply circuit 210, a working power supply circuit 220, a plurality of photoelectric isolation drive circuits 300, a plurality of magnetic isolation power supply circuits 400 and a pulse switch circuit 500.

As shown in FIG. 1, the pulse switch circuit 500 includes a plurality of power switches 510 connected in series between the high-voltage power supply circuit 210 and the ground. The pulse switch circuit 500 is configured to generate and output a high-voltage pulse signal V1 by turning on or off a plurality of power switches 510 based on a driving voltage provided by the high-voltage power supply circuit 210. The photoelectric isolation drive circuits 300 are equal in number to the power switches 510 and correspond to the power switches 510 in a one-to-one corresponding relationship. Each photoelectric isolation drive circuit 300 is individually connected between a corresponding power switch 510 and the control circuit 100. The photoelectric isolation drive circuit 300 is configured to control the turning on or off of the corresponding power switch 510 according to a received switch control signal output by the control circuit 100. The photoelectric isolation drive circuit 300 can isolate the pulse switch circuit 500 from the control circuit 100. The magnetic isolation power supply circuits 400 are equal in number to the optoelectronic isolation drive circuits 300 and correspond to the optoelectronic isolation drive circuits 300 in a one-to-one corresponding relationship. Each magnetic isolation power supply circuit 400 is individually connected to a corresponding optoelectronic isolation drive circuit 300, and each magnetic isolation power supply circuit 400 is connected in series between the working power supply circuit 220 and the ground terminal. The magnetic isolation power supply circuit 400 is configured to supply power to the corresponding optoelectronic isolation drive circuit 300 based on the working voltage provided by the working power supply circuit 220.

This embodiment can achieve isolation among the power switch 510, the high-voltage power supply circuit 210, the working power supply circuit 220 and the control circuit 100 through the above-mentioned photoelectric isolation drive circuit 300 and the magnetic isolation power supply circuit 400, especially isolation between the high-voltage power supply circuit 210 and other circuits, so that each power switch 510 can be controlled independently without being affected by the high-voltage power supply circuit 210. Furthermore, the photoelectric isolation drive circuit 300 has a faster response speed. The photoelectric isolation drive circuit 300 controls the power switch 510 according to the switch control signal, it can improve the synchronization rate among respective power switches 510 while achieving electrical isolation.

Specifically, as shown in FIG. 1, among the plurality of the power switches 510 in the pulse switch circuit 500, the power switches 510 in the number of X are sequentially connected in series to form a first switch branch 520, and the remaining power switches 510 in the number of Y are sequentially connected in series to form a second switch branch 530. A first end of the first switch branch 520 is connected to the high-voltage power supply circuit 210, a second end of the first switch branch 520 is connected to a first end of the second switch branch 530, and a second end of the second switch branch 530 is connected to the ground terminal. The control circuit 100 can synchronously control each power switch 510 in the first switch branch 520 to be turned on or off and can synchronously control each power switch 510 in the second switch branch 530 to be turned on or off. For example, by controlling each power switch 510 in the first switch branch 520 to be turned on or off continually and simultaneously, and controlling each power switch 510 in the second switch branch 530 to be turned off at the same time when the first switch branch 520 is turned on, and controlling each power switch 510 in the second switch branch 530 to be turned on at the same time when the first switch branch 520 is turned off, a corresponding high-voltage pulse signal V1 is generated at the second end of the first switch branch 520. X and Y are both natural numbers greater than 0, and the specific values of X and Y can be determined according to actual conditions. X and Y can be equal. In one embodiment, X and Y are both 3. The power switch 510 can be a Metal-Oxide-Semiconductor Field-Effect Transistor (MOSFET) or an Insulated Gate Bipolar Transistor (IGBT). For example, in this embodiment, an NMOS transistor can be used, as shown in FIG. 1, one of the power switches 510 is an NMOS transistor Q1. The first conducting end of the power switch 510 corresponds to the drain of the NMOS transistor, the second conducting end of the power switch 510 corresponds to the source of the NMOS transistor, and the control end of the power switch 510 corresponds to the source of the NMOS transistor.

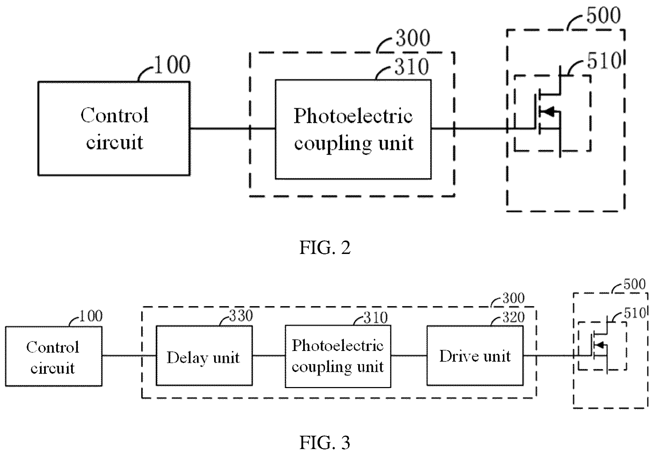

As shown in FIG. 2, in this embodiment, each photoelectric isolation drive circuit 300 includes a photoelectric coupling unit 310. The photoelectric coupling units 310 are equal in number to the power switches 510 and correspond to the power switches 510 in a one-to-one corresponding relationship. The input end of each photoelectric coupling unit 310 can be connected to the control circuit 100, and the output end of each photoelectric coupling unit 310 can be connected to the corresponding power switch 510. The photoelectric coupling unit 310 is configured to unidirectionally transmit the switch control signal to the corresponding power switch 510 through photoelectric conversion.

Specifically, in this embodiment, a total number of X+Y photoelectric isolation drive circuits 300 and a total number of X+Y photoelectric coupling units 310 are provided. The photoelectric coupling unit 310 may be a photoelectric coupler. After receiving the switch control signal and performing photoelectric conversion on the switch control signal, the photoelectric coupling unit 310 can transmit the switch control signal to the corresponding power switch 510 to control the turn-on or turn-off of the power switch 510. While realizing the synchronous control of the power switches 510, the photoelectric coupling units 310 can also isolate the control circuit 100 and the power switches 510 to prevent the driving voltage from being transmitted to the control circuit 100 and affecting the normal operation of the control circuit 100 and the power switch 510.

As shown in FIG. 3, in another embodiment, each photoelectric isolation drive circuit 300 further includes a plurality of drive units 320. The drive units 320 are equal in number to the photoelectric coupling units 310 and correspond to the photoelectric coupling units 310 in a one-to-one corresponding relationship. Each drive unit 320 is individually connected between the output end of the corresponding photoelectric coupling unit 310 (i.e., the photoelectric coupling unit 310 in the same photoelectric isolation drive circuit 300) and the corresponding power switch 510. The drive unit 320 is configured to output a corresponding voltage level to the corresponding power switch 510 according to a switch control signal output by the photoelectric coupling unit 310 to control the turn-on or turn-off of the power switch 510.

It should be noted that, since the output power of the control circuit 100 is low, accordingly the output power of the photoelectric coupling unit 310 is also low, which may not be enough to control the power switch 510 to be turned on or off. Therefore, the drive unit 320 can output a corresponding voltage level according to the control switch signal to control the power switch 510 to be turned on or off. The drive unit 320 can be a MOSFET driver chip, and the MOSFET driver chip can output a corresponding voltage level to drive the power switch 510 according to the switch control signal output by the photoelectric coupling unit 310.

As shown in FIG. 3, in another embodiment, each photoelectric isolation drive circuit 300 further includes a plurality of delay units 330. The delay units 330 are equal in number to the photoelectric coupling unit 310 and correspond to the photoelectric coupling unit 310 in a one-to-one corresponding relationship. Each delay unit 330 is individually connected between the input end of the corresponding photoelectric coupling unit 310 (i.e., the photoelectric coupling unit 310 in the same photoelectric isolation drive circuit 300) and the control circuit 100, so as to adjust the time for the switch control signal to be transmitted to the photoelectric coupling unit 310. Specifically, the delay unit 330 can be an RC delay circuit.

It should be noted that the control circuit 100 is connected to each photoelectric isolation drive circuit 300 through a transmission line, but due to the different lengths of the transmission lines or the different times at which different interfaces of the control circuit 100 output the switch control signal, the transmission times of the switch control signals from the control circuit 100 to respective photoelectric isolation drive circuits 300 are different, which easily leads to the inability of each power switch 510 to be turned on or off synchronously. By providing the corresponding delay unit 330 and adjusting the time for the switch control signal to be transmitted to the photoelectric coupling unit 310, the synchronous control of the power switch 510 can be achieved. For example, the shorter the length of the transmission line between the photoelectric isolation drive circuit 300 and the control circuit 100, the longer the delay time of the delay unit 330 of the photoelectric isolation drive circuit 300, as a result, the times for the switch control signals to be transmitted to respective photoelectric coupling units 310 are the same.

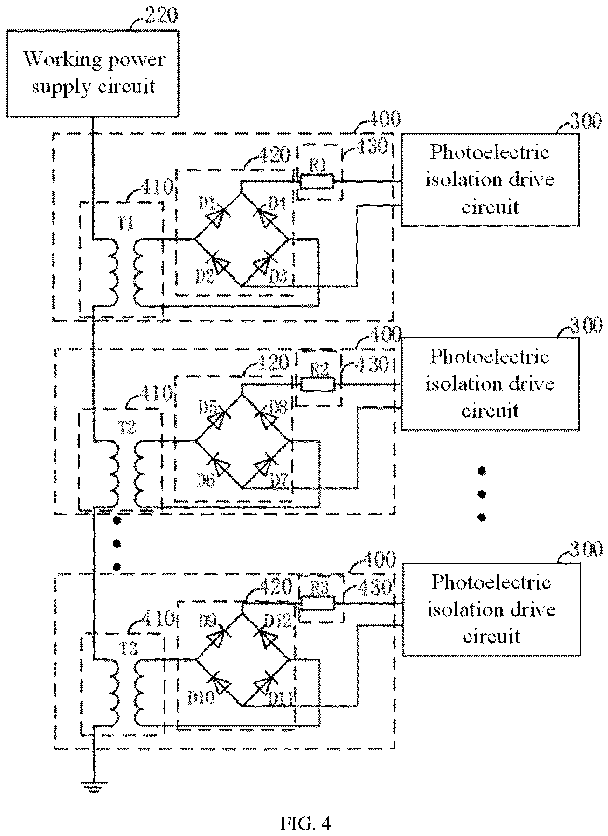

As shown in FIG. 1 and FIG. 4, in this embodiment, each magnetic isolation power supply circuit 400 includes a transformer 410 and a rectifier unit 420. The transformers 410 are equal in number to the photoelectric coupling units 310, and correspond to the photoelectric coupling units 310 in a one-to-one corresponding relationship. The rectifier units 420 are equal in number to the photoelectric coupling units 310, and correspond to the photoelectric coupling units 310 in a one-to-one corresponding relationship. The primary winding of the transformer 410 of each magnetic isolation power supply circuit 400 is connected in series between the working power supply circuit 220 and the ground terminal. The secondary winding of each transformer 410 is individually connected to the corresponding rectifier unit 420 (i.e., the rectifier unit 420 in the same magnetic isolation power supply circuit 400). Each rectifier unit 420 is individually connected to the corresponding photoelectric isolation drive circuit 300 to provide the corresponding working voltage to the corresponding photoelectric isolation drive circuit 300 based on the driving Alternating Current (AC) provided by the working power supply circuit 220.

The transformer 410 and the rectifier unit 420 are only used to provide the working voltage for the corresponding photoelectric isolation drive circuit 300. Although the transformer 410 has a problem of low consistency, it only needs to ensure that the photoelectric isolation drive circuit 300 can work normally, which will not affect the normal turn-off of the power switch 510. At the same time, the photoelectric isolation drive circuit 300 is powered by the magnetic isolation power supply circuit 400, so that the photoelectric isolation drive circuits 300 of different power switches 510 can be isolated from each other.

Specifically, one of the rectifier units 420 includes a first diode D1, a second diode D2, a third diode D3 and a fourth diode D4. The anode of the first diode D1 is connected to the first end of the secondary winding of the transformer 410, and the cathode of the first diode D1 is connected to the working voltage terminal. The anode of the second diode D2, as the ground terminal, is connected to the corresponding photoelectric isolation drive circuit 300, and the cathode of the second diode D2 is connected to the first end of the secondary winding. The anode of the third diode D3 is connected to the anode of the second diode D2, and the cathode of the third diode D3 is connected to the second end of the secondary winding of the transformer 410. The anode of the fourth diode D4 is connected to the second end of the secondary winding of the transformer 410, and the cathode of the fourth diode D4 is connected to the working voltage terminal. The working voltage terminal is configured to connect to the corresponding photoelectric isolation drive circuit 300 to provide a working voltage for the corresponding photoelectric isolation drive circuit 300. While the rectifier unit 420 realizes rectification, the ground terminals of respective rectifier units 420 are independent of each other, so that respective magnetic isolation power supply circuits 400 are not connected to the same reference ground and are isolated from each other. At the same time, the photoelectric isolation drive circuits 300 of different power switches 510 are in a floating ground isolation state, and respective power switches 510 do not interfere with each other.

Among them, between each rectifier unit 420 and the corresponding working voltage terminal, a voltage regulating unit 430 is further provided, and the voltage regulating unit 430 is configured to adjust the voltage output to the photoelectric isolation drive circuit 300. As shown in FIG. 4, specifically, one of the voltage regulating units 430 includes a voltage regulating resistor R1.

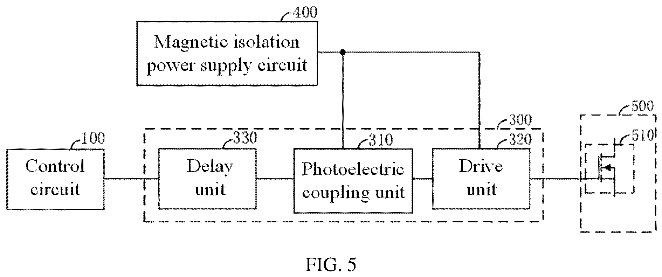

In one example, as shown in FIG. 5, the magnetic isolation power supply circuit 400 may be connected to the photoelectric coupling unit 310 and the drive unit 320 in the corresponding photoelectric isolation drive circuit 300, to supply power to the photoelectric coupling unit 310 and the drive unit 320.

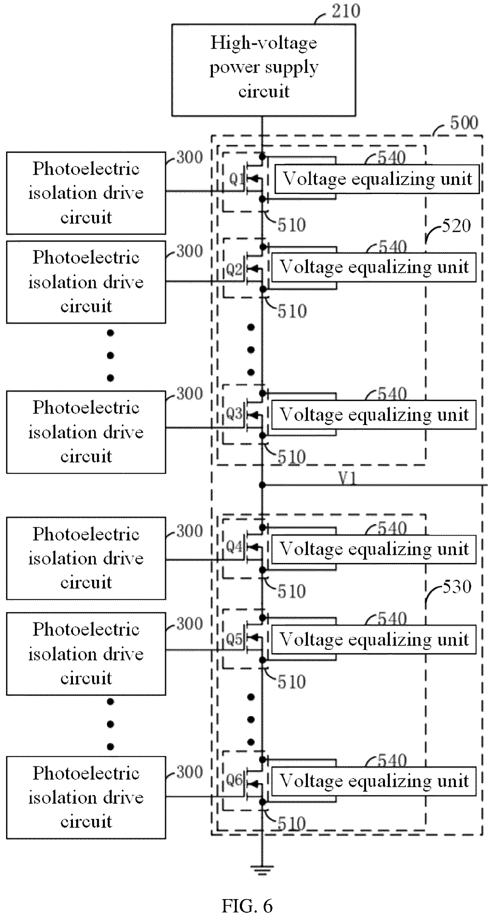

As shown in FIG. 6, in this embodiment, the pulse switch circuit 500 further includes a plurality of voltage equalizing circuits 540. The voltage equalizing circuits 540 are equal in number to the power switches 510 and correspond to the power switches 510 in a one-to-one corresponding relationship. Each voltage equalizing circuit 540 is individually connected in parallel with the corresponding power switch 510 to adjust the voltage across each power switch 510.

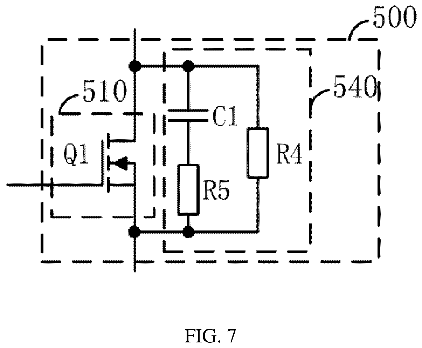

In this embodiment, the voltage equalizing circuit 540 includes a static voltage equalizing resistor. The first end of the static voltage equalizing resistor is connected to the first conducting end of the corresponding power switch 510, and the second end of the static voltage equalizing resistor is connected to the second conducting end of the corresponding power switch 510. When respective power switches 510 are all turned off, the voltages across each respective power switches 510 are equalized by the respective static voltage equalizing resistor. Specifically, as shown in FIG. 7, one of the voltage equalizing circuits 540 includes a resistor R4, which is a static voltage equalizing resistor. The first end of the resistor R4 is connected to the first conducting end of the power switch 510, and the second end of the resistor R4 is connected to the second conducting end of the power switch 510.

In this embodiment, the voltage equalizing circuit 540 further includes a dynamic voltage equalizing resistor and a dynamic voltage equalizing capacitor. The first end of the dynamic voltage equalizing resistor is connected to the first conducting end of the corresponding power switch 510, the second end of the dynamic voltage equalizing resistor is connected to the first end of the dynamic voltage equalizing capacitor, and the second end of the dynamic voltage equalizing capacitor is connected to the second conducting end of the corresponding power switch 510. When the pulse switch circuit 500 generates a corresponding high-voltage pulse signal, the voltages on respective power switches 510 on the same switch branch can be kept equal through the respective dynamic voltage equalizing resistors and the respective dynamic voltage equalizing capacitors. Specifically, as shown in FIG. 7, one of the voltage equalizing circuits 540 includes a resistor R5 and a capacitor C1. The resistor R5 is the dynamic voltage equalizing resistor, and the capacitor C1 is the dynamic voltage equalizing capacitor. The first end of the resistor R5 is connected to the first conducting end of the power switch 510, the second end of the resistor R5 is connected to the first end of the capacitor C1, and the second end of the capacitor C1 is connected to the second conducting end of the power switch 510.

It should be noted that, during the operation of the pulse switch circuit 500, since the plurality of power switches 510 are sequentially connected in series, the parameters such as the parasitic parameters of the power switches 510 may cause uneven voltage division and uneven heating on the respective power switches 510, which may cause thermal breakdown of some power switches 510. By controlling the voltage across each power switch 510 through the voltage equalizing circuit 540, the failure rate of the power switch 510 can be reduced and the service life can be increased.

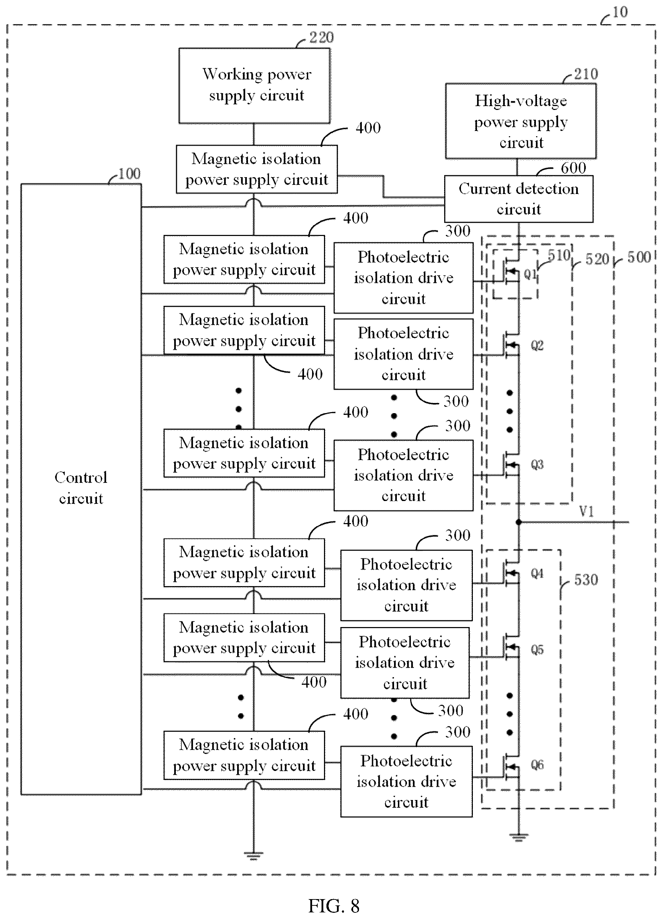

As shown in FIG. 8, in another embodiment, the pulse generation circuit 10 further includes a current detection circuit 600, which is connected between the high-voltage power supply circuit 210 and the pulse switch circuit 500 and is connected to the control circuit 100. The current detection circuit 600 is configured to detect the current output from the high-voltage power supply circuit 210 to the pulse switch circuit 500, generate a corresponding current sampling signal and feed it back to the control circuit 100. The control circuit 100 is connected to the high-voltage power supply circuit 210 and is configured to control the high-voltage power supply circuit 210 to be turned off when the current sampling signal exceeds a preset safety range.

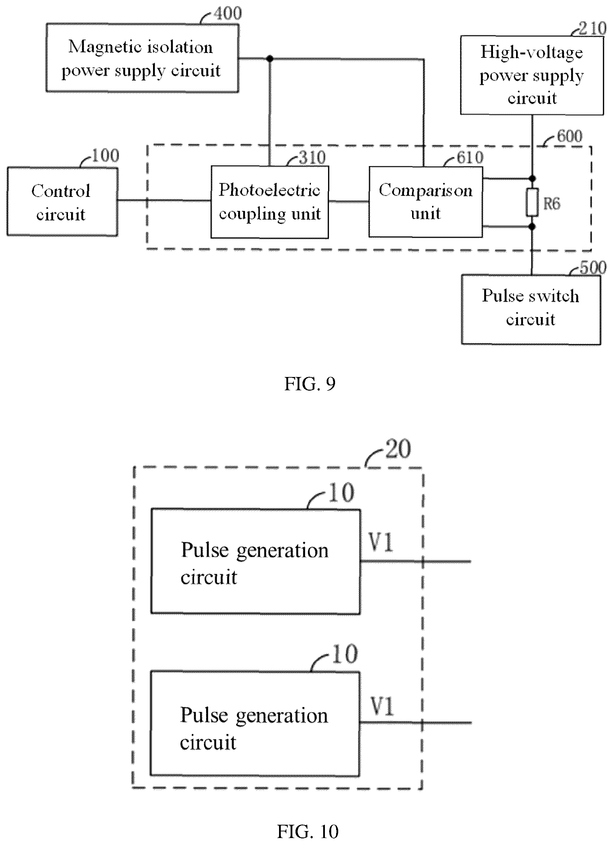

As shown in FIG. 9, specifically, the current detection circuit 600 includes a sampling resistor R6, a comparison unit 610 and a photoelectric coupling unit 310. The sampling resistor R6 is connected in series between the high-voltage power supply circuit 210 and the pulse switch circuit 500, and the comparison unit 610 is respectively connected to two ends of the sampling resistor R6, so as to obtain the electrical parameters of the input current flowing through the sampling resistor R6 according to the voltage at two ends of the sampling resistor R6. The comparison unit 610 is connected to the control circuit 100 through the photoelectric coupling unit 310 of the corresponding current detection circuit 600 to feed back the electrical parameters of the sampled input current to the control circuit 100. Specifically, the comparison unit 610 can be a differential comparator.

The magnetic isolation power supply circuit 400 further includes a transformer 410 and a rectifier unit 420 corresponding to the current detection circuit 600. The rectifier unit 420 is respectively connected to the comparison unit 610 and the photoelectric coupling unit 310 of the current detection circuit 600, so as to provide working voltage for the comparison unit 610 and the photoelectric coupling unit 310 of the current detection circuit 600. Correspondingly, in the pulse generation circuit 10, the number of the magnetic isolation power supply circuits 400 is more than that of the photoelectric isolation drive circuits 300, and the number of the photoelectric coupling units 310 is more than that of the power switches 510.

In another embodiment, there are two current detection circuits 600, one of which is connected between the high-voltage power supply circuit 210 and the pulse switch circuit 500 and is connected to the control circuit 100, while the other is connected between the pulse switch circuit 500 and the ground terminal and is connected to the control circuit 100. The input current and output current of the pulse switch circuit 500 are detected respectively by the two current detection circuits 600, so that the working state of the pulse switch circuit 500 can be known more accurately.

In this embodiment, a thermally conductive ceramic is fixed on the package of each power switch 510 to enhance the heat dissipation of the power switch 510. A temperature detection module is also fixed on the thermally conductive ceramic. The temperature detection module is electrically connected to the control circuit 100. Specifically, the temperature detection module can be a temperature detection sensor or a temperature control switch. The control circuit 100 can monitor the temperature of the thermally conductive ceramic through the temperature detection module, and when it detects that the temperature of the thermally conductive ceramic is too high, each power switch 510 of the pulse switch circuit 500 is controlled to be turned off through the photoelectric isolation drive circuit 300, thereby realizing over-temperature protection of the pulse switch circuit 500.

FIG. 10 shows a schematic diagram of the principle of a pulse generator provided in the second embodiment of the present application. For convenience of explanation, only the part related to this embodiment is shown, which is described in detail as follows:

A pulse generator 20 includes two pulse generation circuits 10 as described in any of the above embodiments, and is used to output two high-voltage pulse signals V1 with interlaced phases through the two pulse generation circuits 10 under the control of a control circuit 100, that is, when one of the high-voltage pulse signals V1 is at a high level, the other high-voltage pulse signal is at a low level, thereby generating a bipolar high-voltage pulse signal.

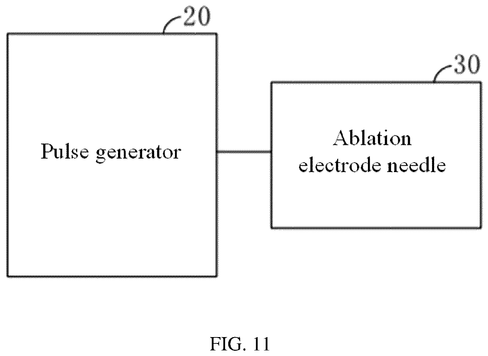

FIG. 11 shows a schematic diagram of the principle of a medical device provided in the third embodiment of the present application. For convenience of explanation, only the part related to this embodiment is shown, which is described in detail as follows:

A medical device includes an ablation electrode needle 30, and further includes a pulse generator 20 as described in any of the above embodiments. The ablation electrode needle 30 is connected to the pulse generator 20 to release a bipolar high-voltage pulse signal. Specifically, in this embodiment, the ablation electrode needle 30 includes a first electrode needle and a second electrode needle to output a bipolar high-voltage pulse signal.

The skilled persons in the art can clearly understand that for the convenience and simplicity of description, the division of the above-mentioned functional units and modules is only used as an example. In actual applications, the above-mentioned function can be allocated to different functional units and modules according to needs. That is, the internal structure of the device can be divided into different functional units or modules to complete all or part of the functions described above. The functional units and modules in the embodiment can be integrated into one processing unit, or each unit can exist physically alone, or two or more units can be integrated into one unit. The above-mentioned integrated unit can be implemented in the form of hardware or in the form of software functional units. In addition, the specific names of the functional units and modules are only for the convenience of distinguishing each other and are not used to limit the scope of protection of the present application. For the specific working process of the units and modules in the above-mentioned system, please refer to the corresponding process in the above-mentioned method embodiment, which will not be repeated here.

In the above-mentioned embodiments, the description of each embodiment has its own emphasis. For the part that is not described or recorded in detail in a certain embodiment, please refer to the relevant description of other embodiments.

The embodiments described above are only used to illustrate the technical solutions of the present application but are not intended to limit them. Although the present application has been described in detail with reference to the aforementioned embodiments, a person skilled in the art should understand that they can still modify the technical solutions described in the aforementioned embodiments, or some of the technical features may be replaced by equivalents. Such modifications or replacements do not cause the essence of the corresponding technical solutions to deviate from the spirit and scope of the technical solutions of the embodiments of the present application and should all be included in the present application and be within the scope of protection.

## Claims

1. A pulse generation circuit comprising: a control circuit, a high-voltage power supply circuit, a working power supply circuit a pulse switch circuit, a plurality of photoelectric isolation drive circuits and a plurality of magnetic isolation power supply circuits,
wherein the pulse switch circuit comprises a plurality of power switches connected in series between the high-voltage power supply circuit and a ground terminal, and the pulse switch circuit is configured to generate and output a high-voltage pulse signal by turning on or off the plurality of the power switches based on a driving voltage provided by the high-voltage power supply circuit;
wherein the photoelectric isolation drive circuits are equal in number to the power switches and correspond to the power switches in a one-to-one corresponding relationship, each of the photoelectric isolation drive circuits is individually connected between a corresponding power switch and the control circuit, and the photoelectric isolation drive circuit is configured to control the turning on or off of the corresponding power switch according to a received switch control signal output by the control circuit; and
wherein the magnetic isolation power supply circuits are equal in number to the photoelectric isolation drive circuits and correspond to the photoelectric isolation drive circuits in a one-to-one corresponding relationship, each of the magnetic isolation power supply circuits is individually connected to a corresponding photoelectric isolation drive circuit, each of the magnetic isolation power supply circuits is connected in series between the working power supply circuit and the ground terminal, and the magnetic isolation power supply circuit is configured to supply power to the corresponding photoelectric isolation drive circuit based on a working voltage provided by the working power supply circuit.

2. The pulse generation circuit of claim 1, wherein each of the photoelectric isolation drive circuits comprises a photoelectric coupling unit, the photoelectric coupling units are equal in number to the power switches and correspond to the power switches in a one-to-one corresponding relationship, an input end of each of the photoelectric coupling units is connected to the control circuit, an output end of each of the photoelectric coupling units is individually connected to the corresponding power switch, and each photoelectric coupling unit is configured to unidirectionally transmit the switch control signal to the corresponding power switch through photoelectric conversion.

3. The pulse generation circuit of claim 2, wherein each of the photoelectric isolation drive circuits further comprises a drive unit, the driving units are equal in number to the photoelectric coupling units and correspond to the photoelectric coupling units in a one-to-one corresponding relationship, each of the drive units is individually connected between the output end of a corresponding photoelectric coupling unit and the corresponding power switch, and each drive unit is configured to output a corresponding voltage level to the corresponding power switch according to the switch control signal output by the corresponding photoelectric coupling unit to control the turning on or off of the power switch.

4. The pulse generation circuit of claim 2, wherein each of the photoelectric isolation drive circuits further comprises a plurality of delay units, the delay units are equal in number to the photoelectric coupling units and correspond to the photoelectric coupling units in a one-to-one corresponding relationship, and each of the delay units is individually connected between the input end of a corresponding photoelectric coupling unit and the control circuit to adjust a time for the switch control signal to be transmitted to the photoelectric coupling unit.

5. The pulse generation circuit of claim 2, wherein each of the magnetic isolation power supply circuits comprises a transformer and a rectifier unit, the transformers are equal in number to the photoelectric coupling units and correspond to the photoelectric coupling units in a one-to-one corresponding relationship, the rectifier units are equal in number to the photoelectric coupling units and correspond to the photoelectric coupling units in a one-to-one corresponding relationship, a primary winding of each transformer is connected in series between the working power supply circuit and the ground terminal, and a secondary winding of each transformer is individually connected to a corresponding rectifier unit, and each rectifier unit is individually connected to the corresponding photoelectric isolation drive circuit to output the corresponding working voltage to the corresponding photoelectric isolation drive circuit.

6. The pulse generation circuit of claim 1, wherein the pulse switch circuit further comprises a plurality of voltage equalizing circuits, the voltage equalizing circuits are equal in number to the power switches and correspond to the power switches in a one-to-one corresponding relationship, and each of the voltage equalizing circuits is individually connected in parallel with the corresponding power switch for adjusting a voltage across a corresponding power switch.

7. The pulse generation circuit of claim 6, wherein each voltage equalizing circuit comprises a static voltage equalizing resistor; a first end of the static voltage equalizing resistor is connected to a first conducting end of the corresponding power switch, and a second end of the static voltage equalizing resistor is connected to a second conducting end of the corresponding power switch.

8. The pulse generation circuit of claim 6, wherein each voltage equalizing circuit comprises a dynamic voltage equalizing resistor and a dynamic voltage equalizing capacitor; a first end of the dynamic voltage equalizing resistor is connected to a first conducting end of the corresponding power switch, a second end of the dynamic voltage equalizing resistor is connected to a first end of the dynamic voltage equalizing capacitor, and a second end of the dynamic voltage equalizing capacitor is connected to a second conducting end of the corresponding power switch.

9. The pulse generation circuit of any one of claims 1 to 8, further comprising a current detection circuit,
wherein the current detection circuit is connected between the high-voltage power supply circuit and the pulse switch circuit and is connected to the control circuit, and wherein the current detection circuit is configured to detect a current output from the high-voltage power supply circuit to the pulse switch circuit, generate a current sampling signal and feed it back to the control circuit; and
wherein the control circuit is connected to the high-voltage power supply circuit and is configured to control the high-voltage power supply circuit to be turned off when the current sampling signal exceeds a preset safety range.

10. A pulse generator, comprising two pulse generation circuits of any one of claims 1 to 9, and being used to output a bipolar high-voltage pulse signal through the two pulse generation circuits under control of the control circuit.

11. A medical device, comprising an ablation electrode needle, and a pulse generator of claim 10, wherein the ablation electrode needle is connected to the pulse generator to release the bipolar high-voltage pulse signal.
